Europäisches Patentamt

European Patent Office

Office européen des brevets

(11) Veröffentlichungsnummer: **0 453 818 B1**

(12) **EUROPÄISCHE PATENTSCHRIFT**

(45) Veröffentlichungstag der Patentschrift: **07.12.94**

(21) Anmeldenummer: **91105113.4**

(22) Anmeldetag: **30.03.91**

(51) Int. Cl.5: **C07C 1/26**, C07C 41/24

(54) **Verfahren zur reduktiven Dehydrohalogenierung von Halogenkohlenwasserstoffen und Halogenethern.**

(30) Priorität: **13.04.90 DE 4012007**

(43) Veröffentlichungstag der Anmeldung:
**30.10.91 Patentblatt 91/44**

(45) Bekanntmachung des Hinweises auf die
Patenterteilung:
**07.12.94 Patentblatt 94/49**

(84) Benannte Vertragsstaaten:
**BE DE FR NL**

(56) Entgegenhaltungen:
DD-A- 235 630          DE-A- 3 510 034
DE-A- 3 715 751        DE-C- 2 164 074
US-A- 3 006 727        US-A- 4 346 246

SOVIET INVENTIONS ILLUSTRATED, CH Sektion, Woche C25, 30. Juli 1980, Derwent Publications Ltd., London, GB

(73) Patentinhaber: **EC ERDÖLCHEMIE GMBH**
**Alte Strasse 201**
**D-50769 Köln (DE)**

(72) Erfinder: **Stüwe, Arnd, Dr.**
**Berta-von-Suttner-Strasse 34**
**W-5090 Leverkusen (DE)**
Erfinder: **Grub, Joachim, Dr.**
**Ostpreussenallee 10**
**W-4047 Dormagen (DE)**
Erfinder: **Lux, Mathias**
**Berliner Ring 5**
**W-5010 Bergheim (DE)**

(74) Vertreter: **Zobel, Manfred, Dr. et al**
**BAYER AG**
**Konzernverwaltung RP**
**Patente Konzern**
**D-51368 Leverkusen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur reduktiven Dehydrohalogenierung von Halogenkohlenwasserstoffen und Halogenethern in der Gasphase in Gegenwart von Aktivkohle, wobei Halogen Chlor oder Brom, bevorzugt Chlor bedeutet.

Die umweltfreundliche Entsorgung von als Abfallstoffe anfallenden halogenierten Kohlenwasserstoffen und Ethern hat in den letzten Jahren zunehmend an Bedeutung gewonnen, Technisch interessant sind hierbei die Verbrennung, die Dehydrohalogenierung und die reduktive Dehydrohalogenierung.

Die Verbrennung ist u.a. dadurch gekennzeichnet, daß neben dem Halogenwasserstoff keine weiteren brauchbaren Produkte anfallen. Die Dehydrohalogenierung liefert häufig nur teilweise enthalogenierte, ungesättigte Produkte, deren Toxizität meist über derjenigen der Ausgangsstoffe liegt. Die reduktive Dehydrohalogenierung zeichnet sich dagegen dadurch aus, daß man neben dem Halogenwasserstoff einen völlig halogenfreien Kohlenwasserstoff erhält, der einer Wiederverwendung zugeführt werden kann.

Beispielsweise wird in DE-PS 21 64 074 die reduktive Dehydrochlorierung von 1,2-Dichlorpropan durch Umsetzung mit Wasserstoff an einem Rhodium-$Al_2O_3$-Katalysator beschrieben, wobei hauptsächlich Propan erhalten wird.

Eine reduktive Dehydrohalogenierung kann jedoch auch durch Umsetzung von Halogenkohlenwasserstoffen mit Kohlenwasserstoffen, gegebenenfalls in Gegenwart von Kohlenstoff, in der Flüssigphase durchgeführt werden (EP 0 198 232 A1). Im Falle von 1,2-Dichlorpropan erhält man nach diesem letzteren Verfahren neben Chlorwasserstoff hauptsächlich Propen. Aus den für die Umsetzung benötigten Kohlenwasserstoffen werden wasserstoffärmere Derivate und teilweise Kohlenstoff gebildet. Eine Variante dieses Verfahrens ist die Durchführung in der Gasphase nach DE-OS 35 10 034, die an aktivem Kohlenstoff durchgeführt wird, der bevorzugt bei der Durchführung der Umsetzung an oberflächenaktiven Mineralien oder Oxiden der Elemente der II. bis V. Hauptgruppe des Periodensystems aus dem Umsetzungsgemisch erzeugt wird. Die Gasphasenvariante wird bei 200-600°C durchgeführt.

Das erstgenannte Verfahren ist einmal durch die Verwendung eines teuren Edelmetallkatalysators gekennzeichnet und zum zweiten dadurch, daß ein relativ wertloser gesättigter Kohlenwasserstoff, nämlich Propan, entsteht. Es wird in der Gasphase durchgeführt. Das zweitgenannte Verfahren, welches in der Sumpfphase durchgeführt wird, liefert zwar einen begehrten ungesättigten Kohlenwasserstoff, nämlich Propen im Falle der Umsetzung von 1,2-Dichlorpropan, und kann auch mit einem kostengünstigen Katalysator, nämlich mit Aktivkohle, arbeiten, falls ein solcher Katalysator überhaupt eingesetzt wird, ist jedoch in negativer Weise durch die stets als Kuppelprodukte auftretenden weiteren ungesättigten Kohlenwasserstoffe gekennzeichnet, die eine aufwendige Auftrennung des Produktstroms erforderlich machen.

Es wurde nun gefunden, daß die reduktive Dehydrohalogenierung von Halogenkohlenwasserstoffen oder Halogenethern mit Wasserstoff in der Gasphase auch an Aktivkohle durchgeführt werden kann. Beim Einsatz von aliphatischen Kohlenwasserstoffen oder Ethern mit wenigstens 2 Halogenatomen, bevorzugt wenigstens 2 vicinalen Halogenatomen werden in vorteilhafter und nicht erwarteter Weise wertvolle ungesättigte Kohlenwasserstoffe erhalten.

Durch das weitgehende Fehlen von organischen Kuppelprodukten wird die Aufarbeitung des Produktstroms vereinfacht und damit ebenfalls kostengünstig.

Es wurde ein Verfahren zur reduktiven Dehydrohalogenierung von geradkettigen oder verzweigten, offenkettigen oder cyclischen Alkanen oder Alkenen, von aromatischen oder araliphatischen Kohlenwasserstoffen oder von aliphatischen oder cyclischen Ethern, die ein oder mehrere Halogenatome tragen, wobei Halogen Chlor oder Brom, bevorzugt Chlor bedeutet, gefunden, das dadurch gekennzeichnet ist, daß solche Halogenkohlenwasserstoffe oder Halogenether in der Gasphase mit Wasserstoff in Gegenwart von Aktivkohle bei Temperaturen von 200-700°C unter Bildung von Halogenwasserstoff umgesetzt werden.

Halogenatome sind Chlor- oder Bromatome, die auch im Gemisch miteinander auftreten können, bevorzugt Chloratome allein.

Geradkettige oder verzweigte offenkettige Halogenalkane haben 1-10, bevorzugt 2-6 C-Atome und sind beispielsweise die folgenden:
$CH_{4-n}Cl_n$ (n = 1-4), $C_2H_{6-n}Cl_n$ (n = 1-6), $C_3H_{8-n}Cl_n$ (n = 1-8), $C_4H_{10-n}Cl_n$ (n = 1-10), Chlorpentan, Dichlorpentan, Chlorhexan, Dichlorhexan, Tetrachlorhexan oder Chloroctan sowie die entsprechenden Bromverbindungen.

Geradkettige oder verzweigte offenkettige Halogenalkene haben 2-10, bevorzugt 2-6 C-Atome und sind beispielsweise die folgenden:
$C_2H_{4-n}Cl_n$ (n = 1-4), $C_3H_{6-n}Cl_n$ (n = 1-6), $C_4H_{8-n}Cl_n$ (n = 1-8), Chlorpentene, Chlorhexene oder Chloroctene sowie die entsprechenden Bromverbindungen.

Halogenhaltige Cycloalkane oder Cycloalkene haben 3-8, bevorzugt 3,5 oder 6 C-Atome und sind beispielsweise die folgenden:
Chlorcyclopentan, Dichlorcyclopentan, Tetrachlorcyclopentan, Chlorcyclohexan, Dichlorcyclohexan, Chlorcyclohexan sowie die entsprechenden Brom-verbindungen.

Aromatische und araliphatische Kohlenwasser-stoffe, die Halogenatome tragen, haben 6 bis 12 C-Atome und sind beispielsweise die folgenden: Chlorbenzol, Vinylchlorbenzol, Dichlorbenzol, Trichlorbenzol, Tetrachlorbenzol, Pentachlorbenzol, Hexachlorbenzol, Chlortoluol, Dichlortoluol, Trichlortoluol, Chlorxylol, Dichlorxylol, Tetrachlorxylol, Chlornaphthalin, Chlormethylnaphthalin, Benzyl-chlorid sowie die entsprechenden Bromverbindun-gen.

Halogenhaltige Dialkylether haben 2-10, bevorzugt 4-6 C-Atome und sind beispielsweise die folgenden: 2,2'-Dichlordiethylether, 2,2'-Dichlordipropylether, 2,2'-Dichlordiisopropylether, 2-Chlorpropyl-2'-chlorisopropylether sowie die entsprechen-den Bromverbindungen. Ein cyclischer halogenhal-tiger Ether ist beispielsweise Epichlorhydrin.

Solche halogenhaltigen Kohlenwasserstoffe und Ether fallen bei verschiedenen chemischen Prozessen als Nebenprodukte an und sind als solche dem Fachmann bekannt.

Wichtige und im erfindungsgemäßen Verfahren umsetzbare Halogenverbindungen sind bevorzugt olefinisch ungesättigte Monohalogenverbindungen, vicinale Di- oder Polyhalogen-alkane und vicinale Di- oder Polyhalogencycloalkane sowie Dihalogendialkylether oder Gemische aus diesen; ein besonders wichtiges Einsatzmaterial ist 1,2-Dichlorpropan (DCP), ein besonders wichtiges Einsatzgemisch ist ein Gemisch aus ca. 90 % DCP, 9 % Dichlordiisopropylether, 0,8 % Epichlorhydrin und 0,2 % 1,2,3-Trichlorpropan, welches bei der Synthese von Propylenoxid nach dem Chlorhydrinverfahren anfällt.

Zur Verfahrensdurchführung wird der Halogenkohlenwasserstoff oder Halogenether im Gemisch mit Wasserstoff über einen Aktivkohle-Kontakt geführt. Der Aktivkohle-Kontakt kann hierbei in Rohren oder auf Böden oder in Form einer Kompaktschüttung in einem geeigneten Apparat angeordnet sein. Die hierzu verwendbaren Apparate zeichnen sich durch große Einfachheit aus und sind dem Fachmann als solche bekannt. Als $H_2$-Menge sei beispielsweise eine solche von 0,3-10 Mol $H_2$ pro Halogenäquivalent des umzusetzenden Halogenkohlenwasserstoffs genannt; in bevorzugter Weise werden 0,5-6 Mol $H_2$ pro Halogenäquivalent einge-setzt.

Dem Gemisch aus Halogenkohlenwasserstoff oder Halogenether und Wasserstoff kann weiterhin ein Inertgas, wie Stickstoff oder ein Edelgas, in einer Menge von 0,001-10 Mol, bevorzugt 0,01 bis 5 Mol Inertgas pro Mol $H_2$ zugesetzt werden.

Das Gemisch aus dem Halogenkohlenwasserstoff oder dem Halogenether, dem Wasserstoff und gegebenenfalls dem Inertgas wird mit einer GHSV (Gaseous Hourly Space Velocity) = 50-2000 Liter des genannten Einsatzgasgemisches pro Liter Aktivkohle-Kontakt pro Stunde über diesen Kontakt geführt. Bevorzugt ist eine GHSV von 100-600 $h^{-1}$.

Das erfindungsgemäße Verfahren wird bei einem Druck von 0,5-10 bar, bevorzugt 0,8-3 bar, besonders bevorzugt bei etwa 1 bar, durchgeführt.

Als Aktivkohle-Kontakt eignen sich alle dem Fachmann bekannten Typen wie solche aus Petrolkoks, Ruß und Tierkohle, bevorzugt Norit RKD 3 Spezial, Carboraffin P oder mit $ZnCl_2$, Phosphor-säure oder Wasserstoff aktivierte Tierkohle.

Das vom Dehydrohalogenierungsreaktor abströmende Reaktionsgemisch enthält neben dem Halogenwasserstoff und überschüssigem Wasser-stoff den zum eingesetzten Halogenkohlenwasser-stoff oder Halogenether korrespondierenden Koh-lenwasserstoff neben nur wenigen weiteren Neben-produkten. Sofern der eingesetzte Halogenwasser-stoff oder Halogenether bereits eine olefinische Doppelbindung enthält, bleibt diese im erfindungsgemäßen Verfahren erhalten. Sofern der eingesetzte Halogenkohlenwasserstoff (cyclo)aliphatisch ist und wenigstens 2 Halogenatome enthält, bildet sich der entsprechende olefinisch ungesättigte Kohlenwasserstoff. Der Sauerstoff wird zu Wasser umgesetzt.

Die Aufarbeitung eines solchen Reaktionsgemisches geschieht zunächst durch Abtrennung des entstandenen Halogenwasserstoffs, beispielsweise absorptiv in einem Wasserwäscher unter Bildung der zugehörigen wäßrigen Halogenwasserstoffsäure, die ihrerseits als Wertprodukt dem Verfahren entnommen werden kann. Auch solche Wäscher zur Abtrennung des Halogenwasserstoffs sind dem Fachmann bekannt. Unvollständig umgesetzte Halogenkohlenwasserstoffe oder Halogenether können destillativ abgetrennt und als solche gewonnen oder bevorzugt in das erfindungsgemäße Verfahren als Einsatzstoff zurückgeführt werden.

Für den Fall der Dehydrohalogenierung von beispielsweise 1,2-Dichlorpropan enthält das der Wasserwäsche abströmende Produktgemisch neben überschüssigem Wasserstoff hauptsächlich Propen und Chlorpropene. Dieses Propen ist, gegebenenfalls nach Kondensation und Abtrennung vom Wasserstoff sowie Chlorpropenen, in vielen Umsetzungen direkt als Ausgangsmaterial einsetzbar. Insbesondere ist ein solches vom Wasserstoff und Chlorpropene befreites Propen ohne weitere Behandlung als Ausgangsmaterial zur Herstellung von Propylenoxid nach dem Chlorhydrin-Verfahren einsetzbar. Die Chlorpropene können zur weiteren Umsetzung in die Reaktion zurückgeführt werden.

Beispiele 1 bis 8

In einem beheizbaren Durchflußreaktor war eine Schüttung von 60 g (150 ml Schüttvolumen) Aktivkohle des Typs Norit RKD 3 Spezial angeordnet worden. Die in der Tabelle angegebenen Mengen an flüssigem 1,2-Dichlorpropan wurden gemeinsam mit den angegebenen Mengen Wasserstoff und in Beispiel 1 zusätzlich mit der angegebenen Menge Stickstoff nach Durchlaufen eines Vorwarmers bei den ebenfalls angegebenen Temperaturen über die Aktivkohle-Schüttung geleitet. Das entstandene HCl-Gas wurde aus dem Reaktionsgemisch mit Hilfe von Wasser ausgewaschen; die verbleibenden Gase wurden aufgefangen und gaschromatographisch analysiert. Die Tabelle zeigt die Analysenergebnisse und die errechneten Umsätze und Selektivitäten.

Tabelle  Dehalogenierung von 1,2-Dichlorpropan (DCP) an 60 g (150 ml Schüttvolumen) Aktivkohle

| Nr. | T/°C | Einsätze DCP ml/h | $H_2$ l/h | $N_2$ l/h | Abgas l/h | $\%N_2$ | $\%H_2$ | $\%C_3H_6$ | $\%C_3H_8$ | $\%C_3H_5Cl$ | $\%C_1,C_2,C_4$ | %DCP |
|---|---|---|---|---|---|---|---|---|---|---|---|---|
| 1 | 400 | 33 | ca.10 | ca.37 | 53 | 69,7 | 15,4 | 2,8 | 0,01 | 8,0 | 0,09 | 4,0 |
| 2 | 420 | 33 | ca.26 | - | 32 | - | 74,4 | 5,7 | 0,18 | 13,0 | 0,6 | 5,8 |
| 3 | 450 | 33 | ca.57 | - | 63 | - | 88,4 | 2,3 | 0,07 | 7,1 | 0,26 | 1,92 |
| 4 | 450 | 16,5 | ca.18 | - | 20 | - | 79,8 | 8,0 | 0,49 | 9,8 | 1,64 | 0,08 |
| 5 | 500 | 16,5 | ca.19 | - | 20 | - | 76,4 | 11,3 | 0,8 | 5,2 | 6,22 | 0,13 |
| 6 | 500 | 16,5 | ca.18 | - | 20 | - | 81,7 | 5,0 | 0,32 | 9,5 | 3,35 | 0,05 |
| 7 | 550 | 16,5 | ca.23 | - | 23 | - | 84,0 | 5,0 | 0,3 | 1,6 | 8,91 | 0,09 |
| 8 | 550 | 33 | ca.27 | - | 27 | - | 49,3 | 22,0 | 5,6 | 1,5 | 21,16 | 0,00 |

## Tabelle (Fortsetzung)

| Nr. | HCl % bezogen auf umgesetztes DCP | HCl g/h | Umsatz DCP % | Ausbeute an KW und Cl-KW bez. auf umges. DCP % | Selektivitäten | | | |
|---|---|---|---|---|---|---|---|---|
| | | | | | %$C_3H_6$ | %$C_3H_8$ | %$C_3H_5Cl$ | %$C_1,C_2,C_4$, als $C_3$ berechnet |
| 1 | 45,8 | 8 | 72 | 107,5 | 25,7 | 0,1 | 73,4 | 0,8 |
| 2 | 58,0 | 10,6 | 75 | 109,3 | 29,3 | 1,2 | 68,3 | 1,2 |
| 3 | 47,0 | 9,6 | 84 | 95,2 | 23,8 | 1,3 | 73,8 | 1,3 |
| 4 | 72,3 | 8,8 | 99,6 | 104,4 | 42,3 | 1,9 | 51,9 | 3,8 |
| 5 | 83,6 | 10,1 | 99,3 | 108,8 | 55,6 | 3,7 | 25,9 | 14,8 |
| 6 | 81,2 | 9,9 | 99,7 | 86,3 | 30,2 | 2,3 | 58,1 | 9,3 |
| 7 | 96,6 | 11,7 | 99,4 | 70,4 | 42,9 | 2,9 | 14,3 | 40,0 |
| 8 | 90,5 | 22,0 | 100,0 | 138,0 | 58,0 | 14,5 | 3,6 | 23,9 |

## Patentansprüche

1. Verfahren zur reduktiven Dehydrohalogenierung von geradkettigen oder verzweigten, offenkettigen oder cyclischen Alkanen oder Alkenen, von aromatischen oder araliphatischen Kohlenwasserstoffen oder von aliphatischen oder cyclischen Ethern, die ein oder mehrere Halogenatome tragen, oder Gemische aus diesen, wobei Halogen Chlor oder Brom, bevorzugt Chlor bedeutet, dadurch gekennzeichnet, daß solche Halogenkohlenwasserstoffe oder Halogenether in der Gasphase mit Wasserstoff in Gegenwart von Aktivkohle bei Temperaturen von 200 bis 700 °C unter Bildung von Halogenwasserstoff umgesetzt werden.

2. Verfahren nach Anspruch 1, dadurch gekennzeichnet, daß als Halogenverbindungen olefinisch ungesättigte Monohalogenverbindungen, vicinale Di- oder Polyhalogen-alkane, vicinale Di- oder Polyhalogen-cycloalkane oder Dihalogendialkylether oder Gemische aus diesen umgesetzt werden.

3. Verfahren nach Anspruch 2, dadurch gekennzeichnet, daß als vicinales Dihalogen-alkan 1,2-Dichlorpropan umgesetzt wird.

4. Verfahren nach Anspruch 1, dadurch gekennzeichnet, daß die Umsetzung bei einem Druck von 0,5-10 bar, bevorzugt von 0,8-3 bar, besonders bevorzugt bei etwa 1 bar durchgeführt wird.

5. Verfahren nach Anspruch 1, dadurch gekennzeichnet, daß der entstandene Halogenwasserstoff durch Absorption in Wasser abgetrennt wird.

6. Verfahren nach Anspruch 1, dadurch gekennzeichnet, daß man nicht umgesetzte oder als Zwischenprodukte entstandene Halogenkohlenwasserstoffe nach destillativer Abtrennung vom halogenwasserstofffreien Produktstrom in die Dehydrohalogenierung zurückführt.

## Claims

1. Process for the reductive dehydrohalogenation of straight-chain or branched, open-chain or cyclic alkanes or alkenes, of aromatic or araliphatic hydrocarbons or of aliphatic or cyclic ethers, which bear one or more halogen atoms, or mixtures of these, with halogen being chlorine or bromine, preferably chlorine, characterized in that such halogenated hydrocarbons or halogenated ethers are reacted in the gas phase with hydrogen in the presence of activated carbon at temperatures of from 200 to 700 °C with formation of hydrogen halide.

**2.** Process according to Claim 1, characterized in that the halogenated compounds reacted are olefinically unsaturated monohalogeno compounds, vicinal di- or polyhalogeno-alkanes, vicinal di- or polyhalogeno-cycloalkanes or dihalogeno-dialkyl ethers or mixtures of these.

**3.** Process according to Claim 1, characterized in that the vicinal dihalogeno-alkane reacted is 1,2-dichloropropane.

**4.** Process according to Claim 1, characterized in that the reaction is carried out at a pressure of 0.5-10 bar, preferably of 0.8-3 bar, particularly preferably at about 1 bar.

**5.** Process according to Claim 1, characterized in that the hydrogen halide formed is separated off by absorption in water.

**6.** Process according to Claim 1, characterized in that unreacted halogenated hydrocarbons or halogenated hydrocarbons formed as intermediates are recirculated into the dehydrohalogenation after the hydrogen halide-free product stream has been separated off by distillation.

**Revendications**

**1.** Procédé de déshalogénhydratation (élimination d'halogénure d'hydrogène) réductrice d'alcanes ou d'alcènes linéaires ou ramifiés, à chaîne ouverte ou cyclique, d'hydrocarbures aromatiques ou araliphatiques ou d'éthers aliphatiques ou cycliques, qui portent un ou plusieurs atomes d'halogène, ou de mélanges de ces composés, le terme halogène désignant le chlore ou brome et de préférence le chlore, procédé caractérisé en ce qu'on fait réagir de tels hydrocarbures halogénés ou de tels éthers halogénés, en phase gazeuse, avec de l'hydrogène, en présence de charbon actif, à des températures de 200 à 700°C avec formation d'un halogénure d'hydrogène.

**2.** Procédé selon la revendication 1, caractérisé en ce qu'on utilise comme composés halogénés des composés monohalogénés à insaturation oléfinique, des di- ou poly-halogéno-alcanes vicinaux, des di- ou poly-halogéno-cycloalcanes vicinaux ou des éthers de dihalogénodialkyle ou des mélanges de ces composés.

**3.** Procédé selon la revendication 2, caractérisé en ce qu'on fait réagir comme dihalogénoalcane vicinal le 1,2-dichloropropane.

**4.** Procédé selon la revendication 1, caractérisé en ce qu'on effectue la réaction sous une pression de 0,5 à10 bar, de préférence de 0,8 à 3 bar et, de façon particulièrement préférée, sous une pression d'environ 1 bar.

**5.** Procédé selon la revendication 1, caractérisé en ce que l'halogénure d'hydrogène résultant est séparé par absorption dans de l'eau.

**6.** Procédé selon la revendication 1, caractérisé en ce qu'on recycle dans la réaction de déshalogénhydratation les hydrocarbures halogénés n'ayant pas réagi ou obtenus comme produits intermédiaires, après en avoir séparé par distillation le courant des produits débarrassés de l'halogénure d'hydrogène.